(19) Europäisches Patentamt
European Patent Office
Office européen des brevets

(11) Publication number: 0 002 908
B1

(12) EUROPEAN PATENT SPECIFICATION

(45) Date of publication of patent specification: 12.05.82

(51) Int. Cl.³: C 07 C 47/19, B 01 J 31/02, C 07 C 45/50

(21) Application number: 78300820.4

(22) Date of filing: 14.12.78

(54) **Hydroformylation of formaldehyde with rhodium catalyst.**

(30) Priority: 16.12.77 US 861474

(43) Date of publication of application:
11.07.79 Bulletin 79/14

(45) Publication of the grant of the patent:
12.05.82 Bulletin 82/19

(84) Designated Contracting States:
BE CH DE FR GB LU NL SE

(56) References cited:
DE - A - 3 940 432
DE - A - 2 741 589
GB - A - 1 335 531
US - A - 3 940 432

Chemical Abstracts vol. 87, no. 19, 1977, Columbus, Ohio, USA
T. MIZOROKI "Syntheses of oxygen-containing $C_2$-compounds using carbon monoxide"
page 541, column 1, abstract no. 151 634g

(73) Proprietor: MONSANTO COMPANY
Patent Department 800 North Lindbergh Boulevard
St. Louis, Missouri 63166 (US)

(72) Inventor: Spencer, Alwyn
Johnson Matthey Research Centre Blount's Court
Jonning Common Reading R64-9NH (GB)

(74) Representative: Lunt, John Cooper et al,
Monsanto House 10-18 Victoria Street
London, SW1H ONQ (GB)

EP 0 002 908 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Hydroformylation of formaldehyde with rhodium catalyst

This invention concerns the production of glycol aldehyde, and in particular a process of preparing glycol aldehyde by reaction of formaldehyde, carbon monoxide and hydrogen in the presence of a rhodium catalyst.

Glycol aldehyde is a known intermediate, and can, for example, be converted to ethylene glycol by hydrogenation. Glycol aldehyde has been prepared by hydroformylation of formaldehyde in the presence of cobalt catalysts as described in United States Patent No. 3,920,753, but the yields reported there do not exceed 50%, based on formaldehyde charged.

The process of the present invention is a process of producing glycol aldehyde which comprises reacting formaldehyde, carbon monoxide and hydrogen at elevated temperature and elevated pressure of carbon monoxide and hydrogen to produce glycol aldehyde in the presence of a metal catalyst system, the reaction being carried out in a solvent in which the reactants and the catalyst system are soluble, characterised in that the metal catalyst system is a rhodium catalyst system and in that the temperature, pressure, solvent and catalyst are selected to favour glycol aldehyde production so that it predominates over methanol production in terms of relative percentage yields.

The rhodium catalyst is of the type suitable for homogeneous hydroformylation reactions. Phosphines or other complexing ligands which have the effect of stabilizing the catalyst against reduction by the formaldehyde are preferably present. Preferred solvents are polar solvents which favour glycol aldehyde production, such as amide solvents. The presence of the glycol aldehyde product has an inhibiting effect upon the reaction, thereby making it advantageous to conduct the process so as to limit the conversion, or to remove product from the reaction mixture.

The use of rhodium catalysts as described herein permits the hydroformylation of formaldehyde to be carried out with good reaction rates and high selectivity to glycol aldehyde.

The reaction catalyzed by rhodium catalyst in the present process can be represented:

$$HCHO + H_2 + CO \rightarrow CH_2OH—CHO$$

It will be recognized that the illustrated reactants are capable of reactions other than that illustrated, and that, depending upon conditions and catalyst, there will be concomitant production of other product in variable amounts, particularly of methanol. However, the conditions in the present process are regulated to give high selectivity to the desired glycol aldehyde.

The present process is carried out under general conditions suitable for hydroformylation with rhodium catalyst. Thus the reaction is effected at elevated temperature and elevated pressure of carbon monoxide and hydrogen. However, relatively mild temperature and pressure conditions are suitable. In fact, there is advantage in not employing too high a temperature, as higher temperatures tend to promote methanol production. The temperature and pressure will generally be sufficiently high to give substantial reaction rates with the catalyst employed, and will usually be at least 50°C. and 35.1 kg./cm.$^2$ (500 psi), and may range up to 200°C. or higher and 351 kg./cm.$^2$ (5000 psi). There is ordinarily no reason for use of higher pressures than this and such use entails higher costs. In order to limit methanol production it is often advantageous to operate at temperature no greater than about 120°C. or even slightly less than that value, but substantial yields of glycol aldehyde can be obtained in the range of about 90° to about 150°C. Pressures in the range 70.3 to 210.9 kg./cm.$^2$ (about 1000 to about 3000 psi) can conveniently be employed. Increasing pressures may promote methanol production to some extent while also influencing reaction rate, but pressures can be selected to give the best balance of rate and selectivity to glycol aldehyde. The temperature, pressure and other conditions are selected so as to have glycol aldehyde production predominate over methanol production, and preferably to have very high selectivity to glycol aldehyde, such as over 90 or over 95, based on the mol ratio of glycol aldehyde to methanol. The methanol produced in the reaction is a useful product. Nevertheless, its production in the present process is ordinarily undesirable and to be minimized as it is generally less valuable than the starting formaldehyde. Aside from other potential uses, the methanol produced can, if desired, be converted to formaldehyde for recycle.

While the CO and $H_2$ react in 1:1 ratio in accordance with the illustrative equation above, it is not necessary to have them present in such ratio for the reaction. As indicated hereinabove, the reactants will be employed at sufficient pressure to provide a suitable reaction rate. The carbon monoxide also contributes to stability of catalyst with carbonyl ligand. The carbon monoxide and hydrogen may conveniently be used in about 1:1 ratio, on a mole basis, as available in synthesis gas, but can also be employed in widely varying ranges, such as CO:$H_2$ mole ratios varying from about 5:95 to 95:5. Good yields are obtainable at CO:$H_2$ ratios as high as 4:1. Large excesses of hydrogen tend to promote the production of methanol, and there is often advantage in having at least an equimolar amount of carbon monoxide, particularly if temperature or other conditions are such as to present a need to repress methanol production.

Rhodium catalyst systems as utilized in the present invention are well known in homogeneous catalysis chemistry, particularly as catalysts for various carboxylation reactions. Generally the rhodium

component of such catalysts is considered to be present in the form of a coordination compound. In addition to the rhodium such coordination compounds can include carbon monoxide ligands, hydride ligands, halide or pseudohalide components, or various other ligands. The term "coordination compound" as used herein means a compound or complex formed by a combination of one or more electronically rich molecules or atoms capable of independent existence with one or more electronically poor molecules each of which may also be capable of independent existence. The rhodium may be complexed with from 3 to 6 or so ligands, and such ligands can and preferably do include halide or pseudohalide ligands, particularly chloride, bromide or iodide. The rhodium can be in form usually considered as neutral or essentially non-valent in common catalyst systems, or in cationic form as described in Tinker and Morris U.S. Patent No. 4,052,461. The catalyst can be supplied to the reaction medium in active form, or in the form of various catalyst precursors and the catalyst may further undergo changes in the course of the reaction, or from the effect of the reaction conditions. In particular it will be recognized that carbon monoxide may replace one or more other ligands upon subjection to carbon monoxide pressures, and further that the carbon monoxide can contribute to stability of the active form of catalyst. There is generally advantage in having catalysts with a halide or pseudohalide component as well as the rhodium component, particularly chlorine, bromine or iodine, as such components contribute to activity, and also affect selectivity to the desired glycol aldehyde, although the chloride ion is considerably better for selectivity than the other halides.

A particular class of rhodium catalysts or precursors which are generally suitable can be represented:

$$Rh\,X\,(CO)\,(P\,L_3)_2$$

where X is Cl, Br, I or F, and $PL_3$ is a phosphine ligand in which each L is an organo group, such as aromatic or alkyl groups, with or without non-interfering substituents. As is often true in rhodium catalyzed reactions, aryl ligands are generally preferred, and such groups may be hydrocarbyl groups with only alkyl or similar substituents, e.g. tolyl, ethylphenyl, etc. although other substituents can be present in the phenyl ring, e.g. fluoro, dimethylamino etc. One or more alkyl groups, e.g. methyl, ethyl, butyl, etc. can be present on the phosphorus, although activity is generally poorer than with phenyl and similar groups.

The rhodium component of the catalyst system employed in the present invention may be provided by introducing into the reaction zone a coordination compound of rhodium, or the rhodium component may be introduced without coordinating ligands. Among the materials which may be charged are rhodium metal, rhodium salts or oxides, organo rhodium compounds, coordination compounds and the like. Specific examples of materials capable of providing the rhodium constituent may be taken from the following non-limiting list of materials:

$RhCl_3$
$RhBr_3$
$RhI_3$
$RhCl_3 . 3H_2O$
$RhBr_3 . 3H_2O$
$Rh_2(CO)_4Cl_2$
$Rh_2(CO)_4Br_2$
$Rh_2(CO)_4I_2$
$Rh_4(CO)_{12}$
$Rh[(C_6H_5)_3P](CO)I$
$Rh[(C_6H_5)_3P]_2(CO)I$
$Rh[(C_6H_5)_3P]_2(CO)Cl$
Rh metal
$Rh(NO_3)_3$
$RhCl[(C_6H_5)_3P]_2(CH_3I)]_2$
$Rh(SnCl_3)[(C_6H_5)_3P]_3$
$RhCl(CO)[(C_6H_5)_3As]_2$
$RhI(CO)[(C_6H_5)_3Sb]_2$
$[(n\text{-}C_4H_9)_4N][Rh(CO)_2X_2]$ where $X=Cl^-, Br^-, I^-$
$[(n\text{-}C_4H_9)_4As][Rh(Co)_2Y_4]$ where $Y=Br^-, I^-$
$[(n\text{-}C_4H_9)_4P][Rh(CO)I_4]$
$Rh[(C_6H_5)_3P]_2(CO)Br$
$Rh[n\text{-}C_4H_9)_3P]_2(CO)Br$
$Rh[(n\text{-}C_4H_9)_3P]_2(CO)I$
$RhBr[(C_6H_5)_3P]_3$
$RhI[(C_6H_5)_3P]_3$
$RhCl[(C_6H_5)_3P]_3$
$RhCl[(C_6H_5)_3P]H_2$

$[(C_6H_5)_3P]_3Rh(CO)H$
$Rh_2O_3$
$[Rh(C_2H_4)_2Cl]_2$
$K_4Rh_2Cl_2(SnCl_3)_4$
$K_4Rh\text{-}Br_2(SnBr_3)_4$

While the catalyst can be represented by formula, it will be recognized that the exact active form is not necessarily known, and various active forms may be present and change during the course of the reaction. Whether the rhodium is provided in an apparently active form, or as a precursor compound, it is to be understood that the invention includes carrying out the process in the presence of rhodium in active catalyst form. In the catalysts where preference is shown for halide anion, pseudohalides can generally be employed in place of halides, although with some variation in results. In some instances pseudohalides will be better than the poorer members of the halides. Pseudohalides are a recognized class of materials having halide like properties in forming co-ordinate compounds and the like, including such members as $NCS^-$, $NCO^-$, $CN^-$, $NCSe^-$, $N_3^-$,

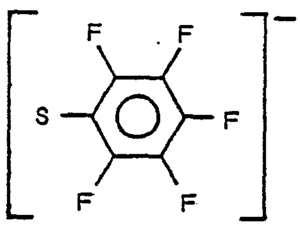
and
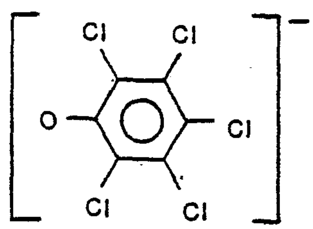

In the present invention a rhodium catalyst is used which is effective for hydroformylation of formaldehyde to glycol aldehyde. In general the catalysts will be those useful for hydroformylations and those skilled in the art can readily determine those particularly suitable for the present reaction. However it is to be noted that phosphine ligands are more suitable than most other complexing ligands employed in the art for stabilizing and similar purposes. Even so, the phosphines are not essential to operability, as the desired reaction will take place in the absence of phosphines, with such groups as halogen, carbonyl etc. serving as coordinating ligands.

The coordinating ligands, such as phosphines, can if desired be present in excess of the amounts required for coordination in the catalyst formula as ordinarily represented, e.g. an $RhCl(CO)(P(C_6H_5)_3)_2$ catalyst may be employed with additional triphenylphosphine present, such as up to 30 to 40 parts on a mole basis per part rhodium. However, large excesses tend to lower activity of the catalyst for the desired reaction, so ordinarily an excess greater than 10 parts of the phosphine will not be employed.

The concentration of the catalyst can be varied widely, but ordinarily sufficient amounts will be employed to obtain desired rates and acceptable selectivity to glycol aldehyde. Ranges from about $10^{-6}$ to about $10^{-1}$ moles/liter of rhodium or even up to 1 mole/liter, are operative, but preferred ranges are about $1 \times 10^{-3}$ moles/liter up to about $2 \times 10^{-2}$ moles/liter. Over fairly broad ranges, the catalyst concentration will not have a marked effect upon selectivity to glycol aldehyde although having some effect upon reaction rate. However, the catalyst concentration can be lowered to an extent sufficient to cause a decline in selectivity to glycol aldehyde, and it will generally be desirable to employ sufficient catalyst for good selectivity. The desirable amounts of catalyst may vary with the amount of formaldehyde reactant present, but will generally be within the above ranges. While higher amounts of catalyst than specified herein can be employed, the use of such amounts is ordinarily undesirable from an economic standpoint.

The present process involves a reaction of formaldehyde, but the formaldehyde is conveniently introduced into the reaction zone in the form of paraformaldehyde. The formaldehyde can be utilized in any form which will generate formaldehyde under the reaction conditions. Aqueous solutions of formaldehyde can be employed, but selectivity to glycol aldehyde is generally poorer than when paraformaldehyde is utilized.

The present reaction can conveniently be effected in a polar solvent which favors the production of glycol aldehyde over that of methanol. Solvents having multiple-bonds from carbon to other atoms, such as those with carbonyl groups, nitrile groups, etc. are among those which tend to be suitable. Such solvents apparently coordinate with the catalyst in some way so as to favor glycol aldehyde production. Of course, solvents which will react readily with the reactants or otherwise unduly interfere in the reaction are to be avoided, and the solvents will generally be relatively inert organic solvents, aside from the coordinating function.

Some amides have been found to be particularly useful as solvents in the present process and to favor production of glycol aldehyde over that of methanol, the amides being characterized by the absence of free hydrogen on the amido nitrogen atom. N,N-disubstituted amides, i.e. amides with two organo substituents, are useful, the amides being compounds characterized by

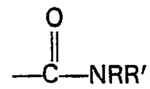

where R and R′ are organo groups, and may usually be hydrocarbyl groups. The substituents on the nitrogen are often alkyl groups, particularly lower alkyl groups such as methyl, ethyl, propyl, butyl, pentyl, isopentyl, hexyl etc. The amides are generally the amides of $C_{1-6}$ carboxylic acids, such as formic, acetic, propionic, hexanoic, etc. There is some variation in selectivity to glycol aldehyde with the variation in the chain length of the acid amide and the substituents on the nitrogen. The acetamides give particularly good results, but there is some loss in selectivity with increasing chain length of the nitrogen substituents on these and other amides. However another factor to be considered is suitability of the solvent for subsequent product separation procedures. As will be discussed further hereinbelow, the reaction of the present invention is not ordinarily carried to high conversions, and it is therefore necessary to separate glycol aldehyde from the formaldehyde reactant. Aqueous extractions have been found to be a suitable separation procedure, and a water immiscible reaction solvent is appropriate for such extraction procedure. The simple amides like dimethylformamide are water soluble but the amides tend toward water immiscibility with increasing chain length, particularly of hydrocarbon moieties, as with alkyl substituents on the nitrogen atom, and immiscibility is reached with sufficiently long chains. Thus di-n-butylformamide and di-isopentylformamide are suitable solvents for aqueous extraction procedures and are also suitable for conducting the hydroformylation reaction. While reaction rates tend to be slower in such solvents than in dimethylformamide etc., the initial rates in the first twenty or thirty minutes of the reaction are good, and in large scale operation it is likely that relatively short reaction times will be utilized with possibly less than maximum conversions in order to increase through-put and enhance selectivity to glycol aldehyde. In order to have a reaction medium which is suitable for both the hydroformylation reaction and the subsequent product separation, it is advantageous to use a water immiscible amide with acceptable properties for the hydroformylation reaction, and the described di-n-butyl and di-isopentylformamides are examples of such water immiscible amides.

The amides useful herein can be compounds with one or more amide groups, and can contain other functional groups so long as such groups do not readily react or unduly interfere in the desired reaction. The amides can be straight chain aliphatic compounds or can be such cyclic compounds as lactams, or contain phenyl or other aromatic groups. For example, such amides as N-methylbutyrolactam and N,N-methylbenzylformamide can be utilized. While the amides can conveniently be used as sole solvent herein, it will be recognized that they will have their desired effect if present in substantial amount even though diluted with other, preferably inert, solvents.

In carrying out the process of the present invention it has been found that good selectivity to glycol aldehyde is obtainable, such as over 90 or 95 based on the ratio of glycol aldehyde to methanol. Moreover, in most of the procedures illustrated herein other potential products or degradation materials were not detected in substantial amounts, so that the high selectivities obtainable are virtually equivalent to yields calculated on formaldehyde reacted.

Another feature of the present process is the fact that the product produced, glycol aldehyde, has an inhibiting effect upon the desired reaction. This indicates it will be advantageous to remove the product from the reaction medium at an early stage by such means as can conveniently be utilized, to take less than complete conversions in batch reactions, or to employ other procedures which may be appropriate. It appears that the aldehyde product may compete with the reactant for catalyst sites, but the applicant is not to be bound by this theory as the inhibiting effect and the procedures described herein have been discovered regardless of what the mechanism may be. Because of the inhibiting effect of the product, it is difficult with the usual concentrations and conditions to obtain conversions based on formaldehyde in batch reactions much above 60% or so. While the use of special conditions, e.g. extremely high catalyst concentrations, would make higher conversions possible, such conditions would have other disadvantages. In general it will be preferable to utilize procedures with lower conversions and to recycle reactant after product separation, or utilize other procedures to limit the concentration of product in the reaction medium. Ordinarily it will be advantageous to operate at conditions for conversions no greater than about 50 or 60%, so that the concentration of glycol aldehyde constitutes no more than 50 or 60% on a molar basis of the glycol aldehyde and formaldehyde present in the reaction medium. If desired, the percentage of product on such basis during the process can be affected by addition of formaldehyde during the reaction, or by removal of a side stream from the reaction medium, and such or similar procedures might be particularly useful in continuous procedures.

A number of the procedures described herein involve reaction times of three hours. However it has been found for many of the illustrated batch conditions, that the initial reaction rate for 20 to 30 minutes is much more rapid than that for the balance of the time. In fact, the bulk of the reaction often occurs during initial stages and the rate falls off as the concentration of glycol aldehyde increases. It follows that a higher through-put and more efficient use of reaction facilities is obtained by shortening the reaction time and taking less than maximum conversions, such as less than 60% or so, and perhaps

even less than 30% or so, based on formaldehyde charged. The use of reaction procedures in which conversions are relatively low, will make the use of efficient product separation procedures particularly advantageous. Glycol aldehyde is difficult to distill because of its reactivity, and successive removal of formaldehyde polymer and other reaction medium components from the glycol aldehyde would be very complex. Therefore the use of a water immiscible reaction solvent, so that the glycol aldehyde can readily be extracted by an aqueous extraction as discussed herein, is particularly advantageous. Thus it is one aspect of the present invention to extract glycol aldehyde by aqueous extraction from a water immiscible amide reaction medium containing substantial amounts of formaldehyde (or its polymers), including such reaction media in which the glycol aldehyde constitutes no more than 50 or 60%, or even no more than 30% of the glycol aldehyde and formaldehyde present. A solvent, despite immiscibility with water, may in the presence of methanol dissolve some water, so it is desirable to have a fairly high degree of immiscibility in order to limit such factors and to permit an efficient extraction.

While a particularly advantageous separation procedure is disclosed herein, it will be recognized that other separation procedures of varying efficiency for the glycol aldehyde product are within the skill of the art.

As disclosed herein, glycol aldehyde is useful as an intermediate for production of ethylene glycol by hydrogenation. As the present process uses hydrogen, ethylene glycol can be produced during or subsequent to the hydroformylation, and the present process includes the production of glycol aldehyde regardless of whether it is recovered as such or converted *in situ* to other products. While in general it may not be desirable to hydrogenate the glycol aldehyde in the presence of formaldehyde reactant and the hydroformylation catalyst, such procedure may be appropriate under particular conditions.

The present process involves a reaction with formaldehyde, which exhibits properties apart from acetaldehyde or aldehydes in general, in being suitable for the described reaction.

As discussed above, a reaction time of three hours is generally more than sufficient in batch reactions, and much shorter times would often be employed. For continuous or semi-batch operations those values can be converted to appropriate values, and the process operated with appropriate space velocity or other measure of reaction zone residence time to obtain the desired degree of conversion, or described maximum ratios of glycol aldehyde product to formaldehyde as discussed hereinabove.

The invention is illustrated by those of the following Examples, where the percentage yield of glycol aldehyde is greater than that of methanol.

Example 1

300 ml stainless steel autoclave equipment with a stirrer, thermocouple and cooling-coil was charged with 100 ml dimethyl formamide, 6 g paraformaldehyde and 0.367 g $RhH(CO)(PPh_3)_3$. The autoclave was sealed and air was removed by flushing the autoclave twice with synthesis gas ($H_2$—CO, 1:1) at 10.5 kg./cm.$^2$ (150 psi gauge). The autoclave and contents were then heated to 110°C. and the pressure was brought to 84.4 kg./cm.$^2$ (1200 psi gauge) with synthesis gas. After 3 hours, the autoclave was cooled to room temperature and the gas phase was released. Gas chromatographic analysis of the liquid phase showed the presence of 1.48 g. glycol aldehyde (12.3% yield) and 0.3 g. methanol (4.7% yield).

In the above example and elsewhere herein, Ph is used as a designation for phenyl, so that, for example $PPh_3$ designates triphenylphosphine. Also occasionally herein dimethylformamide is designated by DMF, dimethylacetamide by DMA, and paraformaldehyde by PF.

Example 2

The procedure of Example 1 was followed, but with 100 ml dimethyl acetamide as solvent. Gas chromatographic analysis showed 0.67 g. glycol aldehyde (5.6% yield) and 0.26 g. methanol (4.1% yield).

Example 3

The procedure of Example 1 was followed, but with 100 ml acetonitrile as solvent. Gas chromatographic analysis showed 0.34 g. glycol aldehyde (2.8% yield); 0.24 g. ethylene glycol (1.9% yield); and 2.75 g. methanol (43% yield).

Example 4

The procedure of Example 1 was employed, but with 100 ml pyridine as solvent. Analysis showed 0.34 g. glycol aldehyde (2.8%); 0.12 g. ethylene glycol (0.8%) and methanol (4.4%).

Example 5

The autoclave of Example 1 was charged with 100 ml dimethyl formamide, 3 g. paraformaldehyde and 0.345 g. $RhCl(CO)(PPh_3)_2$. The reaction was carried out as in Example 1. Gas chromatographic analysis showed 1.41 g. glycol aldehyde (23.5%) and 0.013 g. methanol (0.4%).

Example 6

As in Example 5 at 126.5 kg./cm.² (1800 psig) of a gas blend of carbon monoxide-hydrogen of ratio 4:1 by gas chromatographic analysis showed 2.7 g. glycol aldehyde (46.48%). No methanol was present.

Hydroformylations of formaldehyde were carried out in dimethylformamide with various forms of rhodium catalyst under the same conditions, with resulting yields as reported in Table 1.

TABLE 1

Hydroformylation of Paraformaldehyde with Rhodium Catalysts

| Catalyst | Glycol Aldehyde (%) | Methanol (%) |
|---|---|---|
| $RhCl(CO)(PPh_3)_2$ | 23.5 | 0.4 |
| $[Rh(CO)(PPh_3)_2]BF_4$ | 20.5 | 12.4 |
| $RhCl(CO)_2(PPh_3)$ | 19.6 | 0.4 |
| $Rh_2(CO_2CH_3)_4(PPh_3)_2$ | 9.8 | 5.6 |
| $RhH(CO)(PPh_3)_3$ | 8.5 | 7.8 |
| $Rh(CO_2CH_3)(CO)(PPh_3)_2$ | 8.3 | 20.3 |
| $[Rh(C_8H_{12})(PPh_3)_2]\ BPh_4$ | 3.8 | 26.7 |
| $Rh_4(CO)_{12}$* | 0 | 41.8 |

Catalyst ($5 \times 10^{-3}$ M in Rh) and paraformaldehyde (1M) in dimethylformamide at 110°C. and 84.4 kg./cm.² (1200 psig) $H_2$—CO (1:1). 3 hrs.

* Rhodium metal formed.

Hydroformylations were carried out under comparable conditions, but with variation in reaction temperature, with resulting yields as reported in Table 2.

TABLE 2

Temperature Effect upon Hydroformylation

| Temperature (°C) | Glycol Aldehyde (%) | Methanol (%) |
|---|---|---|
| 95 | 13.9 | 0.1 |
| 110 | 23.5 | 0.4 |
| 120 | 32.0 | 7.8 |
| 125 | 24.3 | 8.8 |
| 140 | 13.8 | 10.3 |
| 170 | 5.6 | 10.3 |

$RhCl(CO)(PPh_3)_2$ ($5 \times 10^{-3}$ M) and paraformaldehyde (1M) in dimethylformamide at 84.4 kg./cm.² (1200 psig). $H_2$—CO (1:1). 3 hrs.

Hydroformylations were carried out under comparable conditions, but with variation in reaction pressure, with resulting yields as reported in Table 3.

TABLE 3

Pressure Effect upon Hydroformylation

| Pressure kg./cm.[2] (psig) | Glycol Aldehyde (%) | Methanol (%) |
|---|---|---|
| 84.4 (1200) | 23.5 | 0.4 |
| 105.4 (1500) | 32.1 | 2.1 |
| 126.5 (1800) | 39.8 | 2.3 |
| 147.6 (2100) | 43.3 | 2.3 |
| 168.7 (2400) | 26.6 | 3.7 |

$RhCl(CO)(PPh_3)_2$ ($5 \times 10^{-3}$M) and paraformaldehyde (1M) in dimethylformamide at 110°C. 3 hrs. $H_2/CO=1$.

Hydroformylations were carried out with $RhX(CO)(PPh_3)_2$ type catalysts in which X represents a designated anion. The hydroformylations were carried out at catalyst concentration of $5 \times 10^{-4}$ mole, with 3 grams paraformaldehyde in 100 ml dimethylformamide at 110°C., 84.4 kg./cm.[2] (1200 psi gauge), $H_2$—CO (1:1) for 3 hours, and resulting yields and selectivity is reported in Table 4. The selectivity (in all Tables herein) is the mole ratio of glycol aldehyde to methanol.

TABLE 4

Hydroformylation with $RhX(CO)(PPh_3)_2$ Types

| X | Glycol Aldehyde (%) | Methanol (%) | Selectivity |
|---|---|---|---|
| $Cl^-$ | 23.5 | 0.4 | 99 |
| $Br^-$ | 19.0 | 7.0 | 73 |
| $I^-$ | 11.0 | 5.0 | 69 |
| $NCS^-$ | 9.6 | 9.0 | 52 |
| $CO_2CH_3^-$ | 8.3 | 20.3 | 29 |
| $F^-$ | 5.7 | 11.3 | 34 |

Catalyst, $5 \times 10^{-3}$M; Paraformaldehyde, 1M; Dimethyl formamide 100 ml; $H_2/CO = 1$; 110°; 3 hrs.

Hydroformylations were carried out with $RhCl(CO)L_2$ type catalyst in which L represents a designated ligand. The conditions were the same as those utilized for Table 4, except the pressure was 126.5 kg./cm.[2] (1800 psi gauge).

TABLE 5

Hydroformylation with $RhCl(CO)(L)_2$ types

| L | Glycol Aldehyde (%) | Methanol (%) | Selectivity |
|---|---|---|---|
| $P(p\text{-}Tol)_3$ | 41.0 | 6.8 | 86 |
| $P(m\text{-}Tol)_3$ | 37.0 | 2.6 | 93 |
| $P(p\text{-}F—C_6H_4)_3$ | 37.0 | 3.8 | 91 |
| $PPh_3$ | 39.8 | 2.3 | 94.5 |
| $P(p\text{-}CH_3OC_6H_4)_3$ | 25.0 | 9.6 | 72 |
| $P(Benzyl)_3$ | 16.0 | 5.0 | 76 |
| $PEtPh_2$ | 11.9 | 5.6 | 68 |
| $P(C_6H_{11})_3$ | 9.0 | 2.5 | 78 |
| $P[p\text{-}(CH_3)_2NC_6H_4]_3$ | 8.2 | 7.8 | 51 |
| $PEt_2Ph$ | 4.6 | 10.0 | 32 |
| $P(n\text{-}C_4H_9)_3$ | 3.7 | 4.8 | 44 |
| $P(o\text{-}Tol)_3$ | 2.2 | 9.0 | 22.6 |
| $P(OC_6H_5)_3$ | 2.0 | 13.0 | 13 |
| $P(C_6F_5)_3$ | 0 | 0 | |
| $AsPh_3$ | 0.5 | 0 | |
| $SbPh_3$ | 0 | 1.2 | |

Catalyst, $5 \times 10^{-3}$M; Paraformaldehyde, 1M; Dimethylformamide, 100 ml; 110°C; 126.5 kg./cm.$^2$ (1800 psig) $H_2$—CO (1:1); 3 hours.

Hydroformylations were carried out in various solvents, under conditions and with results as reported in Table 6.

TABLE 6

Hydroformylation of Paraformaldehyde

| Solvent | Rate kg./cm.$^2$/min (psig/min) | Glycol Aldehyde (%) | Ethylene Glycol (%) | methanol (%) |
|---|---|---|---|---|
| Acetonitrile | 0.28 (3.95) | 2.8 | 1.9 | 43 |
| Acetone | 0.19 (2.75) | 0 | 0 | 42 |
| Benzene | 0.06 (0.80) | 0 | 0 | 19.7 |
| Dioxane | 0.07 (1.01) | 0 | 0 | 31.7 |
| Dimethylformamide | 0.15 (2.08) | 12.3 | 0 | 4.7 |
| Ethanol | 0.41 (5.81) | 0 | 0 | 4.2 |
| Ethylene glycol | 0.06 (0.84) | 0 | 0* | 24 |
| Nitrobenzene | 0.02 (0.27) | 0 | 0 | 6.2 |
| Acetic Acid | 0.14 (1.93) | 0 | 0 | 18.6 |
| DMA | 0.04 (0.64) | 5.6 | 0 | 4.1 |
| Pyridine | 3.58 (50.9) | 2.8 | 0.8 | 4.4 |
| $Ch_3CO_2Et$ | 0.12 (1.66) | 3.8 | 3.5 | 63.6 |
| Tetrahydrofuran | 0.14 (2.00) | 1.5 | 0 | 52.4 |

*No CO uptake.

$RhH(CO)(PPh_3)_3$ ($4 \times 10^{-3}$M) and paraformaldehyde (2M) at 110°C. and 84.4 kg./cm.$^2$ (1200 psig) $H_2$—CO (1:1). 3 hrs.

The results in Table 6 demonstrate some of the effects of solvent and combination of the solvent with the type catalyst employed, a rhodium catalyst with a hydride ligand. The results with the dimethylformamide solvent are poorer than usual results in this solvent with $RhCl(CO)(PPh_3)_2$ catalyst. However the latter catalyst gives inferior results in some solvents, for example producing no glycol aldehyde in acetonitrile when employed with a 1 molar paraformaldehyde at 110° and 1800 psi gauge. Thus glycol aldehyde production may be feasible with a particular solvent with appropriate rhodium catalyst and conditions, despite negative results with the catalyst and conditions of Table 6.

Hydroformylations were carried out with $RhCl(CO)(PPh_3)_2$ in various amides, as reported in Table 7.

TABLE 7

Hydroformylation in Amides

| Solvent | Glycol Aldehyde (%) | Methanol (%) | Selectivity |
|---|---|---|---|
| Formamide | 0 | 0 | — |
| N-Methylformamide | 0 | 0 | — |
| N,N-Dimethylformamide | 39.8 | 2.3 | 94.5 |
| N,N-Diethylformamide | 48.6 | 2.6 | 94.9 |
| N,N-Dipropylformamide | 29.6 | 5.2 | 85.0 |
| N,N-Di-n-butylformamide | 26.6 | 4.7 | 85.0 |
| N-Methyl-N-Phenylformamide | 0 | 0 | — |
| N-Methyl-N-Cyclohexylformamide | 14.7 | 1.8 | 90.2 |
| N-Methyl-N-benzylformamide | 42.7 | 8.6 | 83.2 |
| N,N-dimethylacetamide | 47.7 | 1.2 | 97.6 |
| N,N-dimethylpropionamide | 31.1 | 3.1 | 90.6 |
| N-Methylpyrrolidone | 40.9 | 2.1 | 95.1 |
| N,N-diethylacetamide | 27.2 | 3.8 | 87.7 |

Catalyst $5 \times 10^{-4}$M, Solvent 100 ml; Paraformaldehyde 3 g; 110°C., 133.6 kg./cm.$^2$ (1900 psig) $H_2$—CO (1:1); 3 hours.

The conditions for Table 4 were utilized for hydroformylations with $RhCl(CO)(PPh_3)_2$ catalyst with varying amounts of triphenylphosphine in excess of that in the catalyst formula, as reported in Table 8 where $PPh_3$/Rh represents the mole ratio of the phosphine to rhodium metal.

TABLE 8

Effect of Excess Phosphine

| Triphenylphosphine/ Rhodium | Glycol Aldehyde (%) | Methanol (%) | Selectivity |
|---|---|---|---|
| 1* | 19.6 | 0.3 | 98.5 |
| 2 | 23.5 | 0.4 | 98.3 |
| 5 | 5 | 5 | 50.0 |
| 10 | 4 | 10 | 28.6 |
| 50 | 0 | 14.4 | — |

* $RhCl(CO)_2(PPh_3)$ used as catalyst.

Hydroformylations were carried out utilizing different potential formaldehyde sources, with results as reported in Table 9.

TABLE 9

Formaldehyde Sources

| Formaldehyde Source | Glycol Aldehyde (%) | Methanol (%) | Selectivity |
|---|---|---|---|
| Paraformaldehyde | 23.5 | 0.4 | 98.3 |
| Formalin, 37% | 14.0 | 39.0 | 26.4 |
| Trioxane | 0 | 0 | — |

$RhCl(CO)(PPh_3)_2$, $5 \times 10^{-3}M$; Paraformaldehyde, 1·M; Dimethylformamide, 100 ml; 110°C. 84.4 kg./cm.$^2$ (1200 psig) $H_2$—CO (1:1); 3 hours.

It is apparent that under the reaction conditions the trioxane failed to decompose and provide formaldehyde for the reaction.

Hydroformylations were carried out in which varying amounts of the glycol aldehyde product were added to the initial reaction mixture, with results as reported in Table 10.

TABLE 10

Effect of Added Glycol Aldehyde

| Glycol Aldehyde added (grams) | Glycol Aldehyde* yield (%) | Methanol yield (%) | Selectivity |
|---|---|---|---|
| 0 | 39.8 | 2.3 | 94.5 |
| 1 | 32.5 | 6.0 | 84.4 |
| 2 | 17.2 | 5.0 | 77.5 |
| 3 | 8.3 | 5.6 | 59.7 |

*After Subtraction of GAL added.

$RhCl(CO)(PPh_3)_2$ 0.3453 g.; DMF 100 ml; PF 3 g.; 110°C; 133.6 kg./cm.$^2$ (1900 psig); $H_2$—CO (1:1) 3 hours.

Hydroformylations as carried out with rhodium catalysts with no phosphine ligand, and attempted with several other metal catalysts, are reported in Table 11.

TABLE 11

Hydroformylation of Paraformaldehyde

| Catalyst | Pressure kg./cm.$^2$ (psig) | Glycol Aldehyde (%) | Methanol (%) |
|---|---|---|---|
| $[RhCl(CO)_2]_2$ | 84.4 (1200) | 3.1 | 37.9 |
| $RhCl(CO)_2(C_5H_5N)$ | 133.6 (1900) | 2.9 | 52.9 |
| $IrCl(CO)(PPh_3)_2$ | 133.6 (1900) | 0 | 0 |
| $RuCl_2(PPh_3)_3$ | 133.6 (1900) | 0 | 0 |
| $PtCl_2(PPh_3)_2$ | 133.6 (1900) | 0 | 0 |

Catalyst ($5 \times 10^{-3}$M) and paraformaldehyde (1M) in dimethyl formamide (100 ml) at 110°C. for 3 hours.

Hydroformylations were carried out with varying paraformaldehyde concentration (PF/Rh mole ratio) with results as reported in Table 12.

TABLE 12

Effect of Paraformaldehyde Concentration

| Formaldehyde Rhodium | Glycol Aldehyde (%) | Methanol (%) | Selectivity |
|---|---|---|---|
| 400 | 44.7 | 3.4 | 93.0 |
| 200 | 49.7 | 1.2 | 97.6 |
| 100 | 47.1 | 0 | 100 |
| 50 | 35.0 | 0 | 100 |
| 25 | 26.7 | 0 | 100 |

$PhCl(CO)(PPh_3)_2$ 0.3453 g.; DMA 100 ml.; 133.6 kg./cm.$^2$ (1900 psig) $H_2$—CO (1:1); 3 hours.

Hydroformylations were carried out with varying concentrations of the $Rh(Cl)(CO)(PPh_3)_2$ catalyst, with results as reported in Table 13.

TABLE 13

Variation of Catalyst Concentration

| Formaldehyde/Catalyst (mole ratio) | Glycol Aldehyde (%) | Methanol (%) | Selectivity |
|---|---|---|---|
| 50 | 44.7 | 1.4 | 96.9 |
| 100 | 47.9 | 1.2 | 97.6 |
| 200 | 49.7 | 1.15 | 97.7 |
| 500 | 47.8 | 0.86 | 98.2 |
| 1000 | 26.9 | 2.6 | 91.2 |

DMA 100 ml; PF 3 g.; 110° C.; 133.6 kg./cm.$^2$ (1900 psig) $H_2$—CO (1:1); 3 hours.

Ethylene glycol is a useful commercial commodity and it is one of the objects of the present invention to provide a new route to ethylene glycol. It has been found that glycol aldehyde in solution can be effectively hydrogenated by homogeneous catalysis to ethylene glycol. Hydrogenation procedures and results obtained with particular catalysts at 105.4 kg./cm.$^2$ (1500 psi gauge) hydrogen pressure are set forth in Table 14.

TABLE 14

Hydrogenation of Glycol Aldehyde in Aqueous Solution

| Temp. (C) | Time (h) | Raney * Nickel CON | SEL | 10% Pd on Carbon CON | SEL | 10% Pt on Carbon CON | SEL |
|---|---|---|---|---|---|---|---|
| 150 | 1 | 100 | 13.5 | 92 | 19.2 | 100 | 49.7 |
| 110 | 1 | 100 | 49.4 | 62 | 61 | 100 | 73 |
| 70 | 1 | | | | | 70 | 69.3 |
| 90 | 1 | | | | | 99.4 | 71 |
| 70 | 2 | | | | | 98.3 | 85.6 |
| 50 ** | 3 | 78.8 | 63.7 | 54 | 15.1 | 99 | 74.5 |

* CON = Conversion of glycol aldehyde.
SEL = Selectivity to ethylene glycol.
Glycol aldehyde (1 g) in water (100 ml).
** 70.3 kg./cm.$^2$ (1000 psi gauge).

A copper chromite catalyst at 105.4 kg./cm.$^2$ (1500 psi gauge) hydrogen and 150°C. gave 91% conversion in 1 hour but only 3.2% selectivity to ethylene glycol, while at 110°C. the conversion was 50% with 17.4% selectivity.

The results demonstrate that glycol aldehyde can be catalytically hydrogenated to ethylene glycol. Elevated temperatures and hydrogen pressures can be utilized for such hydrogenation over various known hydrogenation catalysts, with those conditions and catalysts designed for reduction of carbonyl groups to hydroxyl groups being particularly contemplated. The hydrogenation is intended to reduce selectively the aldehyde group in the presence of the hydroxyl group. Various solvents can be used, but the aqueous solutions are particularly useful as the glycol aldehyde is readily soluble therein, and the glycol aldehyde may be recovered in aqueous solution by extraction from the reaction mixture of the hydroformylation process described herein. If desired, benzene and similar hydrocarbon solvents can be used, but it is desirable to use solvents in which substantial amounts of glycol aldehyde can be dissolved.

The results above show a considerable variation in results with various catalysts. The principal byproduct is usually ethanol. It has been found platinum catalyst, particularly platinum on carbon, gives much higher selectivity to ethylene glycol.

**Claims**

1. A process of producing glycol aldehyde which comprises reacting formaldehyde, carbon monoxide and hydrogen at elevated temperature and elevated pressure of carbon monoxide and hydrogen to produce glycol aldehyde in the presence of a metal catalyst system, the reaction being carried out in a solvent in which the reactants and the catalyst system are soluble, characterised in that the metal catalyst system is a rhodium catalyst system and in that the temperature, pressure, solvent and catalyst are selected to favor glycol aldehyde production so that it predominates over methanol production in terms of relative percentage yields.

2. A process of Claim 1 in which the rhodium catalyst system includes a rhodium coordination compound having carbonyl and halide or pseudohalide components.

3. A process of either of Claims 1 and 2 in which the rhodium catalyst system includes a phosphine ligand.

4. A process of Claim 3 in which the ligand is a trisorganophosphine in which the organo groups are aromatic groups and phosphorus is bonded to an aromatic ring carbon.

5. A process of any of Claims 1 to 4 in which an amide solvent is used in which no hydrogen atoms are present on the amide nitrogen.

6. A process of Claim 5 in which the amide is an N,N-dialkylformamide, an N,N-dialkylacetamide or an N,N-dialkylamide of another $C_{1-6}$ alkanoic acid.

7. A process of Claim 6 in which the amide is N,N-dimethylacetamide.

8. A process of Claim 5 in which the amide is water immiscible.

9. A process of any of Claims 1 to 8 in which the catalyst system is represented by $RhX(CO)(PL_3)_2$

in which X is a halide or pseudohalide and $PL_3$ is a phosphine ligand in which each L is an organo group.

10. A process of Claim 9 in which X is chloride.

11. A process of any of Claims 1 to 10 in which the formaldehyde is introduced into the reaction zone as paraformaldehyde.

12. A process of any of Claims 1 to 11 in which the reaction is effected at temperatures in the range of about 50° to about 200°C, pressures of 35.1 to 351 kg./cm.$^2$ (about 500 psi to 5000 psi) and catalyst concentrations of $10^{-6}$ to about $10^{-1}$ moles/liter rhodium.

13. A process of any of Claims 1 to 12 in which the reaction is run to no more than 60% conversion before recovering glycol aldehyde product from the reaction mixture.

14. A process of any of Claims 1 to 13 in which the catalyst, solvent and conditions are selected so as to favor glycol aldehyde production over methanol production, such that the percentage yield of glycol aldehyde is 90% or more of the combined percentage yields of glycol aldehyde and methanol.

15. A process of Claim 8 in which glycol aldehyde is recovered by aqueous extraction.

## Revendications

1. Procédé de production de glycolaldéhyde qui consiste à faire réagir du formaldéhyde, de l'oxyde de carbone et de l'hydrogène à une température élevée et sous une pression élevée d'oxyde de carbone et d'hydrogène pour produire du glycolaldéhyde en présence d'un système catalytique métallique, la réaction étant réalisée dans un solvant où les produits réagissants et le système catalytique sont solubles, caractérisé en ce que le système catalytique métallique est un système de catalyseur au rhodium et en ce que la température, la pression, le solvant et le catalyseur sont choisis pour favoriser la production de glycolaldéhyde afin qu'elle prédomine par rapport à la production de méthanol sous l'angle des rendements relatifs en pourcentage.

2. Procédé selon la revendication 1, dans lequel le système catalytique au rhodium comprend un composé de coordination du rhodium ayant des composants carbonyles et halogénures ou pseudo-halogénures.

3. Procédé selon l'une quelconque des revendications 1 et 2, dans lequel le système catalytique au rhodium comprend un coordinat de phosphine.

4. Composé selon la revendication 3, dans lequel le coordinat est une trisorganophosphine où les groupes organiques sont des groupes aromatiques et le phosphore est lié à un carbone du noyau aromatique.

5. Procédé selon l'une quelconque des revendications 1 à 4, dans lequel on utilise un solvant formé d'amide où aucun atome d'hydrogène n'est présent sur l'azote d'amide.

6. Procédé selon la revendication 5, dans lequel l'amide est une N,N-dialkylformamide, une N,N-dialkylacétamide ou une N,N-dialkylamide d'un autre acide alcanoïque en $C_{1-6}$.

7. Procédé selon la revendication 6, dans lequel l'amide est la N,N-diméthylacétamide.

8. Procédé selon la revendication 5, dans lequel l'amide est non miscible dans l'eau.

9. Procédé selon l'une quelconque des revendications 1 à 8, dans lequel le système catalytique est représenté par $RhX(CO)(PL_3)_2$ où X est un halogénure ou un pseudo-halogénure et $PL_3$ est un coordinat de phosphine où chaque L est un groupe organique.

10. Procédé selon la revendication 9, dans lequel X est le chlorure.

11. Procédé selon l'une quelconque des revendications 1 à 10, dans lequel le formaldéhyde est introduit dans la zone de réaction sous forme de paraformaldéhyde.

12. Procédé selon l'une quelconque des revendications 1 à 11, dans lequel la réaction est effectuée à des températures dans l'intervalle d'environ 50 à environ 200°C, sous des pressions de 35,1 à 351 kg/cm$^2$ et avec des concentrations de catalyseur de $10^{-6}$ à environ $10^{-1}$ mole/litre de rhodium.

13. Procédé selon l'une quelconque des revendications 1 à 12, dans lequel le réaction est réalisée jusqu'à une conversion non supérieure à 60% avant de récupérer le glycolaldéhyde produit à partir du mélange réactionnel.

14. Procédé selon l'une quelconque des revendications 1 à 13, dans lequel le catalyseur, le solvant et les conditions sont choisis afin de favoriser la production de glycolaldéhyde par rapport à la production de méthanol, de manière telle que le pourcentage de rendement de glycolaldéhyde soit 90% où plus des rendements combinés en pourcentage de glycolaldéhyde et de méthanol.

15. Procédé selon la revendication 8, dans lequel le glycolaldéhyde est récupéré par extraction aqueuse.

## Patentansprüche

1. Verfahren zur Herstellung von Glykolaldehyd durch Umsetzen von Formaldehyd, Kohlenmonoxid und Wasserstoff bei erhöhter Temperatur und erhöhtem Druck von Kohlenmonoxid und Wasserstoff unter Bildung von Glykolaldehyd in Gegenwart eines Metallkatalysatorsystems, wobei die Reaktion in einem Lösungsmittel, in dem die Reaktionsteilnehmer und das Katalysatorsystem löslich sind, durchgeführt wird, dadurch gekennzeichnet, daß das Metallkatalysatorsystem ein Rhodium-Katalysatorsystem ist und daß die Temperatur der Druck, das Lösungsmittel und der katalysator derart ausgewählt werden, daß die Glykolaldehyd-Bildung begünstigt wird, so daß sie die Methanol-Bildung im Hinblick auf die relativen prozentualen Ausbeuten überwiegt.

2. Verfahren nach Anspruch 1, dadurch gekennzeichnet, daß das Rhodium-Katalysatorsystem eine Rhodium-Koordinationsverbindung mit Carbonyl- und Halogenid- oder Pseudohalogenid-Komponenten enthält.

3. Verfahren nach einem der Ansprüche 1 und 2, dadurch gekennzeichnet, daß Rhodium-Katalysatorsystem einen Phosphinliganden enthält.

4. Verfahren nach Anspruch 3, dadurch gekennzeichnet, daß der Ligand ein Trisorganophosphin ist, dessen Organogruppen aromatische Gruppen sind und wobei der Phosphor an ein aromatisches Ringkohlenstoffatom gebunden ist.

5. Verfahren nach einem der Ansprüche 1 bis 4, dadurch gekennzeichnet, daß man ein Amid-Lösungsmittel verwendet, an dessen Amid-Stickstoffatom keine Wasserstoffatome vorliegen.

6. Verfahren nach Anspruch 5, dadurch gekennzeichnet, daß das Amid ein N,N-Dialkylformamid, ein N,N-Dialkylacetamid oder ein N,N-Dialkylamid einer anderen $C_1$—$C_6$-Alkansäure ist.

7. Verfahren nach Anspruch 6, dadurch gekennzeichnet, daß das Amid N,N-Dimethylacetamid ist.

8. Verfahren nach Anspruch 5, dadurch gekennzeichnet, daß das Amid mit Wasser nicht mischbar ist.

9. Verfahren nach einem der Ansprüche 1 bis 8, dadurch gekennzeichnet, daß das Katalysatorsystem der Formel

$$RhX(CO)(PL_3)_2$$

entspricht, in der X für ein Halogenid oder ein Pseudohalogenid und $PL_3$ für einen Phosphinliganden stehen, dessen Gruppe L jeweils eine Organogruppe darstellt.

10. Verfahren nach Anspruch 9, dadurch gekennzeichnet, daß X für Chlorid steht.

11. Verfahren nach einem der Ansprüche 1 bis 10, dadurch gekennzeichnet, daß man den Formaldehyd als Paraformaldehyd in die Reaktionszone einführt.

12. Verfahren nach einem der Ansprüche 1 bis 11, dadurch gekennzeichnet, daß man die Reaktion bei Temperaturen im Bereich von etwa 50 bis etwa 200°C, Drücken von 35,1 bis 351 kg/m$^2$ (etwa 500 psi bis 5000 psi) und Katalysatorkonzentrationen von $10^{-6}$ bis etwa $10^{-1}$ Mol/l Rhodium durchführt.

13. Verfahren nach einem der Ansprüche 1 bis 12, dadurch gekennzeichnet, daß man die Reaktion bis zu einer Umsetzung von nicht mehr als 60% durchführt, bevor man das Glykolaldehydprodukt aus der Reaktionsmischung gewinnt.

14. Verfahren nach einem der Ansprüche 1 bis 13, dadurch gekennzeichnet, daß man den Katalysator, das Lösungsmittel und die Bedingungen derart auswählt, daß die Glykolaldehyd-Bildung gegenüber der Methanol-Bildung begünstigt wird, so daß die prozentuale Glykolaldehyd-Ausbeute 90% oder mehr der kombinierten prozentualen Ausbeuten von Glykolaldehyd und Methanol entspricht.

15. Verfahren nach Anspruch 8, dadurch gekennzeichnet, daß man den Glykolaldehyd durch wäßrige Extraktion gewinnt.